Europäisches Patentamt

**European Patent Office**

Office européen des brevets

(11) Publication number: **0 035 364
B1**

# EUROPEAN PATENT SPECIFICATION

(45) Date of publication of patent specification: **23.05.84**

(51) Int. Cl.³: **A 61 K 7/13, D 06 P 3/08**

(21) Application number: **81300786.1**

(22) Date of filing: **25.02.81**

(54) Aqueous compositions for darkening keratinous materials.

(30) Priority: **28.02.80 GB 8006844**

(43) Date of publication of application:
**09.09.81 Bulletin 81/36**

(45) Publication of the grant of the patent:
**23.05.84 Bulletin 84/21**

(84) Designated Contracting States:
**AT BE CH DE FR GB IT LI NL SE**

(56) References cited:
**FR-A-1 439 307
FR-A-1 498 572
FR-A-2 245 340
US-A-1 937 365**

(73) Proprietor: **COMBE INCORPORATED
240 Westchester Avenue
White Plains New York 10604 (US)**

(72) Inventor: **Holland, David O.
"Lindfield" Keppel Road
Dorking Surrey (GB)**

(74) Representative: **Pennant, Pyers
Stevens, Hewlett & Perkins 5 Quality Court
Chancery Lane
London, WC2A 1HZ (GB)**

Courier Press, Leamington Spa, England.

## Description

Solutions of salts of bismuth have been used to darken human hair and other keratinous matter for many years. For example, British Patent Specification 1467874 provides a hair-dyeing composition comprising sulphur and the water-soluble reaction product of bismuth citrate and triethanolamine in an aqueous vehicle. However, the rate at which these solutions react with the hair to produce the dark colour is slow, and many such formulations are not stable and need to be freshly prepared before use. Sulphur and sodium thiosulphate have been recommended to hasten the darkening process, but the rate at which hair is darkened has nevertheless hitherto been unsatisfactory. Lead salts have also been used in hair darkening compositions; but the same problems arise and in addition the known toxicity of lead is a disadvantage.

It is an object of the present invention to provide aqueous compositions containing bismuth which are able to darken fibrous keratinous materials such as wool and fur and, particularly, hair and which are at the same time stable.

The present invention provides an aqueous composition for darkening fibrous keratinous material comprising a mixture of a solution of a salt of thiosulphuric acid, with a solution of a complex of bismuth with an aminopolyacetic acid, in the presence of a water-soluble amine, and of a di- or poly-hydroxy humectant. Preferably the aqueous composition comprises from 0.2 to 5.0% by weight of thiosulphate ion $(S_2O_3^=)$, from 0.1 to 5.0% by weight of bismuth (as carbonate), water-soluble amine to maintain the pH in the range 8—11 and from 5 to 70% by weight of the humectant.

The amino-polyacetic acid used as a complexing agent for bismuth may suitably be nitrilotriacetic acid (NTA) or ethylenediamine tetracetic acid (EDTA), EDTA being preferred. The method of preparation of the complex is not critical. According to one method, 10 parts of bismuth carbonate or bismuth oxycarbonate or the equivalent quantity of bismuth oxide may be suspended in water with either 12.5 parts of EDTA or 13.6 parts of NTA, the suspension heated, and a water-soluble amine added slowly with stirring until virtually all the solid matter is dissolved.

The composition of the invention preferably contains 0.1 to 5% by weight, particularly 0.25 to 1.5% by weight of bismuth, expressed as carbonate. The amount of complexing agent (e.g. EDTA) used should preferably be no more than is necessary to form a water-soluble complex, since an excess appears to decrease the efficiency of the composition for darkening hair.

While the nature of the water-soluble amine is not critical, we have found that alkanolamines are particularly effective. Among these may be used monoethanolamine, diethanolamine, triethanolamine and tri-isopropanolamine. One of these alkanolamines may be used to complete preparation of the water-soluble bismuth complex as described above. In addition, it is preferred to include a further amount of alkali, preferably a water-soluble amine in an amount from 1—10%, particularly 2—5%, of the composition. The amount of excess alkali used should preferably be such as to bring the final pH of the composition to a value in the range 8—11, preferably 8—10.5. Compositions of the invention preferably have a pH in the range 6—11, particularly 8—10.5.

As a di- or poly-hydroxy humectant, propylene glycol is preferred, although glycerol or polyethylene glycol may be used. The nature and proportion of the humectant materially affects the speed of darkening achieved by the composition. Thus for example, glycerol may be used to produce brown colours rapidly up to a concentration of about 30%. Above that level it has an increasingly deleterious effect. Propylene glycol on the other hand is particularly effective in promoting colour production in the range 20 to 70%.

The water-soluble complexes of bismuth described above have some colouring effect on hair by themselves, but this effect is very much enhanced by the presence of thiosulphate. Compositions of this invention accordingly contain thiosulphate ion in a proportion of 0.2—5%, preferably 1—3% by weight. The thiosulphate may be added as the salt of any inert non-toxic metal, for example as sodium thiosulphate. Ammonium thiosulphate may be used, but is liable to give rise to a smell of ammonia. A preferred alternative is to use the thiosulphate of an amine, such as the amine or alkanolamine described above in connection with the bismuth complex. Amine thiosulphates may readily be prepared by boiling an aqueous mixture of ammonium thiosulphate with an amine having a suitable vapour pressure and water solubility; the resulting aqueous solution can be used without the need to isolate the compound. Solutions of mono-, di- and triethanolamine thiosulphate are particularly convenient to prepare and use in this way. The amine used is additional to that used in forming the water-soluble complex of bismuth and the further proportion included in the composition as described above.

It is possible to include sulphur in the aqueous composition, in addition to the thiosulphate, but this has the disadvantage of incorporating a water-insoluble ingredient. Other conventional ingredients, such as monohydric alcohols, perfumes, thickening agents and surface active agents, may be used in conventional amounts.

The invention also includes a method of darkening fibrous keratinous material by applying to the keratinous material an aqueous composition as described above.

The following examples illustrate the invention. Examples 1 to 5 describe the preparation of water-soluble complexes of bismuth. The remaining examples describe compositions according to the invention.

Example 1

Bismuth carbonate (5.0 g) and ethylenediamine tetracetic acid (6.25 g) are suspended in 120 ml water and the mixture is heated with stirring at not less than 60°C until effervescence ceases. Triethanolamine (about 5.0 g) is then added slowly with continued stirring to produce a solution with a pH of about 8.0. The solution, which contains a small quantity (about 0.01 g) of insoluble residue, is cooled to room temperature and filtered from the insoluble residue. The filter is washed with a little water and the washings added to the main filtrate which is then diluted to 200 ml with water.

Example 2

A mixture of bismuth carbonate (10 g) and ethylenediamine tetracetic acid (12.5 . g) in water (60 ml) is treated as in Example 1, the final filtered solution being diluted to 100 ml. 9.5 g of triethanolamine was used in this preparation and the insoluble residue weighed 0.03 g.

Example 3

Proceed essentially as in Example 1 above, but using 6.8 g of nitrilotriacetic acid in place of the ethylenediamine tetracetic acid.

Example 4

Bismuth carbonate (5.0 g) and ethylenediamine tetra tetracetic acid (6.25 g) are suspended in 60 ml water and the mixture is heated to about 60°C with stirring while monoethanolamine is added dropwise to about pH 6. Heating and stirring are continued until effervescence has virtually ceased and then monoethanolamine is again added until the pH has risen to about 9.7 when nearly all the solid matter will have dissolved. The mixture is then cooled to room temperature and filtered from the insoluble residue (about 0.1 g). The residue is washed as in Example 1 and the final solution is diluted to 100 ml. About 3 g of monoethanolamine was used in this preparation.

Example 5

Proceed essentially as in Example 4 but using diethanolamine instead of monoethanolamine. The final pH is about 9.1.

Example 6

To 10 ml of the solution prepared according to Example 1 there is added, with stirring, a mixture of nonyl phenol ethoxylate (9 mole) (hereafter referred to as NP9) (0.5 g), triethanolamine (3 g), glycerol (15 g) and isopropyl alcohol (6 ml). To this solution is then added a solution of sodium thiosulphate (3 g) in water (20 ml) and the whole is diluted to 100 ml with water. This solution contains 1.37% as thiosulphate ion and has a pH of approximately 9.5.

This composition darkens hair gradually with successive applications.

Example 7

To a mixture of NP9 (0.5 g), triethanolamine (3 g) and isopropyl alcohol (6 ml) and water (10 ml) add 2.5 ml of the solution prepared as described in Example 2. To this mixture then add propylene glycol (60 g) followed by a solution of sodium thiosulphate (5 g) in water (15 ml). Stir the solution and dilute to 100 ml with water. This formula contains 2.26% as thiosulphate ion.

This composition darkens hair significantly with one application.

Example 8

To a solution of NP9 (0.5 g), triethanolamine (3 g), perfume (0.2 g) and glycerol (20 g) in ethyl alcohol (20 ml) add, with stirring, 10 ml of the solution prepared as described in Example 1 followed by a solution of sodium thiosulphate (5 g) in water (20 ml). To the final mixture add a solution of hydroxyethylcellulose (0.1 g) in water (10 ml) and dilute the whole to 100 ml.

Example 9

To a solution of NP9 (0.5 g), triethanolamine (8 g), glycerol (20 g) and isopropyl alcohol (6 ml) in water (20 ml) add, with stirring, 10 ml of the solution prepared as described in Example 1 followed by a solution of sodium thiosulphate (5 g) in water (20 ml). Dilute the resulting mixture with water to 100 ml.

Example 10

Following the procedure described in Example 9, use 10 ml of the solution prepared as described in Example 3 in place of that described in Example 1 and 3 g triethanolamine rather than 8 g. Also before final dilution with water to 100 ml add 2 g finely divided sulphur.

This composition will darken hair at a faster rate than will a similar composition which does not contain sulphur.

Example 11

Follow the procedure described in Example 6, but use 3 g diethanolamine instead of triethanolamine. The approximate pH of the final solution is 11.0.

Example 12

To a mixture of NP9 (0.5 g), isopropyl alcohol (6 ml), triethanolamine (9 g) and propylene glycol (55 g) add, with stirring, 10 ml of the solution prepared as described in Example 1 followed by ammonium thiosulphate (3 g) and dilute the mixture to 100 ml.

This composition acts similarly to that of Example 7.

## Example 13

To a solution of ammonium thiosulphate (30 g) in water (120 ml) add triethanolamine (60 g). Heat the mixture to boiling and continue to boil till the total weight is reduced to about 150 g when most of the ammonia released will have been driven off. The pH of this mixture is about 8.5. To this solution, at room temperature, is then added, with stirring, a mixture comprising 50 ml of the solution prepared as described in Example 2 a polyethanoxyl alkyl ether containing a nominal eight molecules of ethylene oxide (hereafter referred to as KA8 (5 g), triethanolamine (30 g), propylene glycol (400 g) and isopropyl alcohol (60 ml). The whole is then diluted to 1 litre with water. The solution contains 2.3% as thiosulphate ion.

This composition acts similarly to that of Example 7.

## Example 14

Heat a solution of ammonium thiosulphate (30 g) and triethanolamine (60 g) in water (120 ml) as described in Example 13 and dilute the final concentrated mixture to 150 ml. Add an aliquot (15 ml) of this solution with stirring to a mixture of NP9 (0.5 g), isopropyl alcohol (6 ml) and propylene glycol (55 g) and to the resulting solution add 10 ml of the solution prepared as described in Example 1. Dilute the resulting mixture with water to 100 ml.

This composition will darken hair or wool by immersion to a marked degree.

## Example 15

To a mixture of KA8 (0.5 g), triethanolamine (3 g), propylene glycol (45 g) and perfume (0.2 g) in ethanol (12 ml) add, with stirring, 10 ml of the solution prepared as described in Example 2 followed by 15 ml of the solution prepared from ammonium thiosulphate and triethanolamine as described in Example 14.

## Example 16

Heat a mixture of ammonium thiosulphate (10 g) and diethanolamine (14.2 g) in water (10 ml) until the mixture has lost 5 g in weight and dilute the resultant solution to 50 ml with water. To 15 ml of this solution add 3 g triethanolamine and proceed then as in Example 14. The final solution contains 2.3% as thiosulphate ion.

This composition performs similarly to that of Example 7.

## Example 17

Concentrate a mixture of ammonium thiosulphate (10 g) and monoethanolamine (8.24 g) in water (20 ml) by boiling to a final weight of about 21 g. Dilute the concentrate to 50 ml with water and use 15 ml of this solution as described in Example 16. The final mixture contains 2.3% as thiosulphate ion.

This composition performs similarly to that of Example 7.

## Example 18

Proceed as in Example 14, but use 10 ml of the solution prepared as in Example 3 instead of as in Example 1, and include also 3 g triethanolamine.

This composition performs similarly to that of Example 7.

## Example 19

To a mixture of KA8 (0.5 g), diethanolamine (3 g), propylene glycol (50 g) and isopropyl alcohol (6 ml) add 5 ml of the solution prepared as in Example 2. Stir and then add 15 ml of the solution prepared from ammonium thiosulphate and triethanolamine as described in Example 14. This mixture has a pH of about 9.5. It gives very good darkening of the hair with one application.

## Example 20

Proceed as in Example 19 but use monoethanolamine (3 g) in place of diethanolamine. The mixture has a pH of about 10.4 and has a similar action on hair to that of Example 19.

## Example 21

To a mixture of KA8 (0.5 g), diethanolamine (3 g), propylene glycol (50 g) and isopropyl alcohol (6 ml) add 10 ml of the solution prepared as in Example 5. Stir and then add 15 ml of the solution prepared from ammonium thiosulphate and diethanolamine as described in Example 16. This has pH 9.6 and gives particularly good darkening of the hair with one application.

## Example 22

To a mixture of KA8 (0.5 g), monoethanolamine (3 g), propylene glycol (50 g) and isopropyl alcohol (6 ml) add 10 ml of the solution prepared as in Example 4. Stir and then add 15 ml of the solution prepared from ammonium thiosulphate and monoethanolamine as described in Example 17. This has pH 10.5 and gives very good darkening of the hair with one application.

Tests on white wool, grey hair tresses and human volunteers have shown that these compositions will darken hair or wool much more rapidly than formulations heretofore described.

Samples kept for six months in glass containers at 40°C have shown no sign of deterioration and chemical analysis has indicated no change in the thiosulphate.

**Claims for the Contracting States: BE, CH, DE, FR, GB, IT, LI, NL, SE**

1. Aqueous composition for darkening fibrous keratinous material comprising a mixture of a solution of a salt of thiosulphuric acid, with a solution of a complex of bismuth with an aminopolyacetic acid, in the presence of a water-soluble amine, and of a di- or polyhydroxy humectant.

2. Aqueous composition as claimed in claim 1, comprising from 0.2—5.0% by weight of thiosulphate ion ($S_2O_3=$), from 0.1 to 5.0% by weight of bismuth (as carbonate), a water-soluble amine to maintain the pH of the composition in range 8—11, and from 5—70% by weight of the humectant.

3. Aqueous composition as claimed in claim 1 or claim 2, wherein the amine is an alkanolamine.

4. Aqueous composition as claimed in claim 3, wherein the alkanolamine is monoethanolamine, diethanolamine or triethanolamine.

5. Aqueous composition as claimed in any one of claims 1 to 4, wherein the amino-polyacetic acid is nitrilotriacetic acid or ethylenediamine tetracetic acid.

6. Aqueous composition as claimed in any one of claims 1 to 5, wherein the solution of a salt of thiosulphuric acid is provided by a solution of an amine thiosulphate.

7. Aqueous composition as claimed in any one of claims 1 to 5, wherein the solution of a salt of thiosulphuric acid is provided by a solution of sodium, potassium or ammonium thiosulphate.

8. Aqueous composition as claimed in any one of claims 1 to 7, wherein the humectant is propylene glycol.

9. Aqueous composition as claimed in claim 8, wherein the propylene glycol is present in an amount of 20—70% by weight.

10. Aqueous composition as claimed in any one of the claims 1 to 7, wherein the humectant is glycerol.

11. Aqueous composition as claimed in claim 10, wherein the glycerol is present in an amount of 5 to 30% by weight.

12. Aqueous composition as claimed in any one of claims 1 to 11, comprising thiosulphate ion in an amount of 1—3% by weight, bismuth (as carbonate) in an amount of 0.1—1.5% by weight, the bismuth being present in the form of a complex with ethylenediamine tetracetic acid in the presence of monoethanolamine, diethanolamine or triethanolamine in an amount to maintain the pH of the composition in the range 8 to 10.5, and propylene glycol in an amount of 35—65% by weight.

13. A method of darkening fibrous keratinous material which method comprises applying to the keratinous material the aqueous composition claimed in any one of claims 1 to 12.

## Claims for the Contracting State: AT

1. A process for the production of an aqueous composition for darkening fibrous keratinous material which comprises mixing together a solution of a salt of thiosulphuric acid and a solution of a complex of bismuth with an amino-polyacetic acid, in the presence of a water-soluble amine and of a di- or poly-hydroxy humectant.

2. A process as claimed in claim 1, which comprises mixing together from 0.2—5.0% by weight of thiosulphate ion ($S_2O_3=$), from 0.1 to 5.0% by weight of bismuth (as carbonate), a water-soluble amine to maintain the pH of the composition in range 8—11, and from 5—70% by weight of the humectant.

3. A process as claimed in claim 1 or claim 2, wherein the amine component is an alkanolamine.

4. A process as claimed in claim 3, wherein the alkanolamine is monoethanolamine, diethanolamine or triethanolamine.

5. A process as claimed in any one of claims 1 to 4, wherein the amino-polyacetic acid component is nitrilotriacetic acid or ethylenediamine tetracetic acid.

6. A process as claimed in any one of claims 1 to 5, wherein the solution of a salt of thiosulphuric acid is provided by a solution of an amine thiosulphate.

7. A process as claimed in any one of claims 1 to 5, wherein the solution of a salt of thiosulphuric acid is provided by a solution of sodium, potassium or ammonium thiosulphate.

8. A process as claimed in any one of claims 1 to 7, wherein the humectant component is propylene glycol.

9. A process as claimed in claim 8, wherein the propylene glycol is present in an amount of 20—70% by weight.

10. A process as claimed in any one of claims 1 to 7, wherein the humectant component is glycerol.

11. A process as claimed in claim 10, wherein the glycerol is present in an amount of 5 to 30% by weight.

12. A process as claimed in any one of claims 1 to 11, which comprises mixing together thiosulphate ion in an amount of 1—3% by weight, bismuth (as carbonate) in an amount of 0.1—1.5% by weight, the bismuth being present in the form of a complex with ethylenediamine tetracetic acid, in the presence of mono-ethanolamine, diethanolamine or triethanol-amine in an amount to maintain the pH of the composition in the range 8 to 10.5, and propylene glycol in an amount of 35—65% by weight.

13. A method of darkening fibrous keratinous material which method comprises applying to the keratinous material the aqueous composition produced by the process of any one of claims 1 to 12.

## Patentansprüche für die Vertragsstaaten: BE, CH, DE, FR, GB, IT, LI, NL, SE

1. Wässerige Mischung für das Dunkelfärben von faserigem Keratinmaterial, bestehend aus einer Mischung einer Lösung eines Salzes der Thioschwefelsäure mit einer Lösung einers Komplexes von Wismuth mit einer Aminopolyessigsäure in Gegenwart eines wasserlöslichen Amins und eines Di- oder Poly-hydroxy-Befeuchtungsmittels.

2. Wässerige Mischung nach Anspruch 1, bestehend aus 0,2 bis 5,0 Gew.-% Thiosulfation ($S_2O_3{}^{2-}$), 0,1 bis 5,0 Gew.-% Wismuth (als Carbonat), einem wasserlöslichen Amin, um den pH-Wert der Mischung im Bereich von 8 bis 11 zu halten, und 5 bis 70 Gew.-% des Befeuchtungsmittels.

3. Wässerige Mischung nach Anspruch 1 oder Anspruch 2, in welcher das Amin ein Alkanolamin ist.

4. Wässerige Mischung nach Anspruch 3, in welcher das Alkanolamin Monoäthanolamin, Diäthanolamin oder Triäthanolamin ist.

5. Wässerige Mischung nach einem der Ansprüche 1 bis 4, in welcher die Aminopolyessigsäure Nitrilotriessigsäure oder Äthylendiaminetetraessigsäure ist.

6. Wässerige Mischung nach einem der Ansprüche 1 bis 5, in welcher die Lösung eines Salzes der Thioschwefelsäure eine Lösung eines Aminthiosulfats ist.

7. Wässerige Mischung nach einem der Ansprüche 1 bis 5, welche die Lösung eines Salzes der Thioschwefelsäure eine Lösung von Natrium-, Kalium- oder Ammoniumthiosulfat ist.

8. Wässerige Mischung nach einem der Ansprüche 1 bis 7, in welcher das Befeuchtungsmittel Propylenglykol ist.

9. Wässerige Mischung nach Anspruch 8, in welcher das Propylenglykol in einer Menge von 20 bis 70 Gew.-% vorliegt.

10. Wässerige Mischung nach einem der Ansprüche 1 bis 7, worin das Befeuchtungsmittel Glycerin ist.

11. Wässerige Mischung nach Anspruch 10, in welcher das Glycerin in einer Menge von 5 bis 30 Gew.-% vorliegt.

12. Wässerige Mischung nach einem der Ansprüche 1 bis 11, bestehend aus Thiosulfation in einer Menge von 1 bis 3 Gew.-%, Wismuth (als Carbonat) in einer Menge von 0,1 bis 1,5 Gew.-%, wobei das Wismuth in Form eines Komplexes mit Äthylendiamintetraessigsäure vorliegt, in Gegenwart von Monoäthanolamin, Diäthanolamin oder Triäthanolamin in einer Menge, um den pH-Wert der Mischung im Bereich von 8 bis 10,5 zu halten, und Propylenglykol in einer Menge von 35 bis 65 Gew.-%.

13. Verfahren zum Dunkelfärben von faserigem Keratinmaterial, bei welchem auf das Keratinmaterial die wässerige Mischung, wie sie in einem beliebigen der Ansprüche 1 bis 12 beansprucht ist, aufgebracht wird.

**Patentansprüche für den Vertragsstaat: AT**

1. Verfahren zur Herstellung einer wässerigen Mischung für das Dunkelfärben von faserigem Keratinmaterial, dadurch gekennzeichnet, daß eine Lösung eines Salzes der Thioschwefelsäure und eine Lösung eines Wismith-Komplexes mit einer Aminopolyessigsäure in Gegenwart eines wasserlöslichen Amins und eines Di-oder Polyhydroxy-Befeuchtungsmittels vermischt werden.

2. Verfahren nach Anspruch 1, dadurch gekennzeichnet, daß 0,2 bis 5,0 Gew.-% Thiosulfation ($S_2O_3{}^{2-}$), 0,1 bis 5,0 Gew.-% Wismuth (als Carbonat), ein wasserlösliches Amin zur Einstellung des pH-Wertes der Mischung im Bereich von 8 bis 11 und 5 bis 70 Gew.-% des Befeuchtungsmittels miteinander vermischt werden.

3. Verfahren nach Anspruch 1 oder 2, dadurch gekennzeichnet, daß die Aminkomponente ein Alkanolamin ist.

4. Verfahren nach Anspruch 3, dadurch gekennzeichnet, daß das Alkanolamin Monoäthanolamin, Diäthanolamin oder Triäthanolamin ist.

5. Verfahren nach einem der Ansprüche 1 bis 4, dadurch gekennzeichnet, daß die Aminopolyessigsäure - Komponente Nitrilotriessigsäure oder Äthylendiamintetraessigsäure ist.

6. Verfahren nach einem der Ansprüche 1 bis 5, dadurch gekennzeichnet, daß die Lösung eines Salzes der Thioschwefelsäure eine Lösung eines Aminthiosulfats ist.

7. Verfahren nach einem der Ansprüche 1 bis 5, dadurch gekennzeichnet, daß die Lösung eines Salzes der Thioschwefelsäure eine Lösung von Natrium-, Kalium- oder Ammoniumthiosulfat ist.

8. Verfahren nach einem der Ansprüche 1 bis 7, dadurch gekennzeichnet, daß die Befeuchtungskomponente Propylenglykol ist.

9. Verfahren nach Anspruch 8, dadurch gekennzeichnet, daß das Propylenglykol in einer Menge von 20 bis 70 Gew.-% vorliegt.

10. Verfahren nach einem der Ansprüche 1 bis 7, dadurch gekennzeichnet, daß die Befeuchtungskomponente Glycerin ist.

11. Verfahren nach Anspruch 10, dadurch gekennzeichnet, daß das Glycerin in einer Menge von 5 bis 30 Gew.-% vorliegt.

12. Verfahren nach einem der Ansprüche 1 bis 11, dadurch gekennzeichnet, daß Thiosulfation in einer Menge von 1 bis 3 Gew.-% und Wismuth (als Carbonat) in einer Menge von 0,1 bis 1,5 Gew.-%, wobei das Wismuth in Form eines Komplexes mit Äthylendiamintetraessigsäure vorliegt, in Gegenwart von Monoäthanolamin, Diäthanolamin oder Triäthanolamin in einer Menge, um den pH-Wert der Mischung im Bereich von 8 bis 10,5 zu halten, und Propylenglykol in einer Menge von 35 bis 65 Gew.-% zusammengemischt werden.

13. Verfahren zum Dunkelfärben von faserigem Keratinmaterial, dadurch gekennzeichnet, daß auf das Keratinmaterial eine wässerige Mischung, hergestellt gemäß einem oder mehrer der Ansprüche 1 bis 12, aufgebracht wird.

**Revendications pour les Etats Contractants:
BE, CH, DE, FR, GB, IT, LI, NL, SE**

1. Composition aqueuse pour foncer des matières fibreuses kératiniques, caractérisée en ce qu'elle comprend un mélange d'une solution d'un sel d'acide thiosulfurique avec une solution d'un complexe de bismuth formé avec un acide amino-polyacétique, en présence d'une amine hydrosoluble et d'un agent humidifiant dihydroxylé ou polyhydroxylé.

2. Composition aqueuse selon la revendication 1, caractérisée en ce qu'elle comprend entre 0,2 et 5,0% en poids de l'ion thiosulfate $(S_2O_3^=)$ entre 0,1 et 5,0% en poids de bismuth (sous forme de carbonate), une amine hydrosoluble pour maintenir le pH de la composition entre 8 et 11 et entre 5 et 70% en poids de l'agent humidifiant.

3. Composition aqueuse selon la revendication 1 ou 2, caractérisée en ce que l'amine est une alcanolamine.

4. Composition aqueuse selon la revendication 3, caractérisé en ce que l'alcanolamine est la mono-éthanolamine, la diéthanolamine ou la triéthanolamine.

5. Composition aqueuse selon l'une quelconque des revendications 1 à 4, caractérisée en ce que l'acide amino-polyacétique est l'acide nitrilotriacétique ou l'acide éthylènediaminetétracétique.

6. Composition aqueuse selon l'une quelconque des revendications 1 à 5, caractérisée en ce que la solution d'un sel d'acide thiosulfurique est fournie par une solution d'un amino-thiosulfate.

7. Composition aqueuse selon l'une quelconque des revendications 1 à 5, caractérisée en ce que la solution d'un sel d'acide thiosulfurique est fournie par une solution de thiosulfate de sodium, de potassium ou d'ammonium.

8. Composition aqueuse selon l'une quelconque des revendications 1 à 7, caractérisée en ce que l'agent humidifiant est le propylène-glycol.

9. Composition aqueuse selon la revendication 8, caractérisé en ce que le propylène-glycol est présent en une quantité comprise entre 20 et 70% en poids.

10. Composition aqueuse selon l'une quelconque des revendications 1 à 7, caractérisée en ce que l'agent humidifiant est le glycérol.

11. Composition aqueuse selon la revendication 10, caractérisée en ce que le glycérol est présent en une quantité comprise entre 5 et 30% en poids.

12. Composition aqueuse selon l'une quelconque des revendications 1 à 11, caractérisée en ce qu'elle comprend l'ion thiosulfate en une quantité comprise entre 1 et 3% en poids, le bismuth (sous forme de carbonate) en une quantité comprise entre 0,1 et 1,5% en poids, ce bismuth étant présent sous la forme d'un complexe avec l'acide éthylènediaminetétracétique, en présence de mono-éthanolamine, de diéthanolamine ou de triéthanolamine en une quantité suffisante pour maintenir le pH de la composition entre 8 et 10,5, et de propylène-glycol en une quantité comprise entre 35 et 65% en poids.

13. Procédé pour foncer des matières fibreuses kératiniques, caractérisé en ce qu'on applique aux matières kératiniques la composition aqueuse selon l'une quelconque des revendications 1 à 12.

**Revendications pour l'Etat Contractant: AT**

1. Procédé de préparation d'une composition aqueuse pour foncer des matières fibreuses kératiniques, caractérisé en ce qu'on mélange une solution d'un sel d'acide thiosulfurique avec une solution d'un complexe de bismuth formé avec un acide amino-polyacétique, en présence d'une amine hydrosoluble et d'un agent humidifiant dihydroxylé ou polyhydroxylé.

2. Procédé selon la revendication 1, caractérisé en ce qu'on mélange entre 0,2 et 5,0% en poids de l'ion thiosulfate $(S_2O_3^=)$, entre 0,1 et 5,0% en poids de bismuth (sous forme de carbonate), une amine hydrosoluble pour maintenir le pH de la composition entre 8 et 11, et entre 5 et 70% en poids de l'agent humidifiant.

3. Procédé selon la revendication 1 ou 2, caractérisé en ce que l'amine est une alcanolamine.

4. Procédé selon la revendication 3, caractérisé en ce que l'alcanolamine est la mono-éthanolamine, la diéthanolamine ou la tri-éthanolamine.

5. Procédé selon l'une quelconque des revendications 1 à 4, caractérisé en ce que l'acide mono-polyacétique est l'acide nitrilotriacétique ou l'acide éthylènediaminetétracétique.

6. Procédé selon l'une quelconque des revendications 1 à 5, caractérisé en ce que la solution d'un sel d'acide thiosulfurique est fournie par une solution d'un amino-thiosulfate.

7. Procédé selon l'une quelconque des revendications 1 à 5, caractérisé en ce que la solution d'un sel d'acide thiosulfurique est fournie par une solution de thiosulfate de sodium, de potassium ou d'ammonium.

8. Procédé selon l'une quelconque des revendications 1 à 7, caractérisé en ce que l'agent humidifiant est le propylèneglycol.

9. Procédé selon la revendication 8, caractérisé en ce que le propylèneglycol est présent en une quantité comprise entre 20 et 70% en poids.

10. Procédé selon l'une quelconque des revendications 1 à 7, caractérisé en ce que l'agent humidifiant est le glycérol.

11. Procédé selon la revendication 10, caractérisé en ce que le glycérol est présent en une quantité comprise entre 5 et 30% en poids.

12. Procédé selon l'une quelconque des revendications 1 à 11, caractérisé en ce qu'on mélange ensemble l'ion thiosulfate en une quantité comprise entre 1 et 3% en poids, le bismuth (sous forme de carbonate) en une quantité comprise entre 0,1 et 1,5% en poids, ce bismuth étant présent sous la forme d'un complexe avec l'acide éthylènediaminetétracétique, en présence de mono-éthanolamine, de di-éthanolamine ou de tri-éthanolamine en une quantité suffisante pour maintenir le pH de la composition entre 8 et 10,5, et le propylèneglycol en une quantité comprise entre 35 et 65% en poids.

13. Procédé pour foncer des matières fibreuses kératiniques, caractérisé en ce qu'on applique aux matières kératiniques la composition aqueuse selon l'une quelconque des revendications 1 à 12.